Europäisches Patentamt

European Patent Office

Office européen des brevets

(11) Veröffentlichungsnummer: **0 031 895**
**B1**

# EUROPÄISCHE PATENTSCHRIFT

(45) Veröffentlichungstag der Patentschrift:
23.02.83

(21) Anmeldenummer: 80107419.6

(22) Anmeldetag: 27.11.80

(51) Int. Cl.³: **C 07 C 19/045, C 07 C 17/156**

(54) Verfahren zur Herstellung von 1,2-Dichlorethan.

(30) Priorität: 08.12.79 DE 2949530

(43) Veröffentlichungstag der Anmeldung:
15.07.81 Patentblatt 81/28

(45) Bekanntmachung des Hinweises auf die Patenterteilung:
23.02.83 Patentblatt 83/8

(84) Benannte Vertragsstaaten:
BE DE FR GB IT NL

(56) Entgegenhaltungen:
DE-A-2 449 563
DE-B-1 229 999
DE-B-2 232 301

(73) Patentinhaber: HOECHST AKTIENGESELLSCHAFT,
Postfach 80 03 20, D-6230 Frankfurt/Main 80 (DE)

(72) Erfinder: Kühn, Wenzel, Dr., Kampenwandstrasse 15,
D-8261 Burgkirchen/Alz (DE)
Erfinder: Riedl, Josef, Dr., Kantstrasse 53,
D-8261 Burgkirchen/Alz (DE)
Erfinder: Widmann, Peter, Hüttenbergerweg 37a,
D-8262 Altötting (DE)

Verfahren zur Herstellung von 1,2-Dichlorethan

Die Erfindung betrifft ein Verfahren zur Herstellung von 1,2-Dichlorethan gemäss Anspruch 1.

1,2-Dichlorethan wird bereits seit einer Reihe von Jahren in grossem Masse industriell hergestellt. Es wird hauptsächlich durch thermische Spaltung zu Vinylchlorid umgesetzt, welches wiederum die Grundlage für den Massenkunststoff Polyvinylchlorid ist. Diese Verwendung hat 1,2-Dichlorethan zu einem der am meisten produzierten aliphatischen, chlorierten Kohlenwasserstoffe gemacht. Zu seiner Herstellung sind verschiedene Verfahren bekannt, von denen die meisten von Ethylen ausgehen. Im allgemeinen wird an Ethylen direkt elementares Chlor angelagert, wobei man bei Temperaturen von 40 bis etwa 120°C in flüssiger Phase, häufig in 1,2-Dichlorethan, arbeitet. In einer viel gebrauchten Form des Verfahrens wird die bei der Chloranlagerung entstehende beträchtliche Wärmemenge durch siedendes 1,2-Dichlorethan abgeführt.

Bei der thermischen Spaltung des 1,2-Dichlorethans zu Vinylchlorid entsteht Chlorwasserstoff, der wiederum zusammen mit Sauerstoff oder Luft dazu verwendet wird, weiteres Ethylen zu chlorieren. Ein derartiger Oxichlorierungsprozess wird im allgemeinen bei Temperaturen zwischen 180 und 320°C in Gegenwart eines sogenannten Deacon-Katalysators (meist Kupferchlorid mit Zusätzen), der entweder als Festbett oder in Wirbelschicht vorliegt, durchgeführt.

Bisher wurden die Direktchlorierung und die Oxichlorierung von Ethylen in getrennten Apparaten vorgenommen, wohl weil man befürchtete, dass bei den relativ hohen Temperaturen, die eine wirksame Oxichlorierung erfordert, die direkte Umsetzung von Ethylen mit Chlor zu Nebenreaktionen, beispielsweise Substitutionsreaktionen, anstelle einer einfachen Anlagerung führt, und ausserdem der bei der Oxichlorierung entstehende Wasserdampf weitere Störungen und Nebenreaktionen veranlasst. Man zog es vor, die direkte Anlagerung von Chlor an Ethylen bei erheblich niedrigeren Temperaturen und in flüssiger Phase in Gegenwart von vergleichsweise geringen Mengen eines Katalysators (meist Eisen(III)chlorid) vorzunehmen, unter Inkaufnahme des Nachteils, dass die hierbei entstehende erhebliche Wärmemenge auf einem ungünstig niedrigem Temperaturniveau anfällt, eine Tatsache, die ihre wirtschaftliche Nutzung sehr erschwert.

In der US-PS Nr. 3288868 ist eine Methode zur Oxichlorierung aliphatischer Kohlenwasserstoffe und ihrer unvollständig chlorierten Derivate in einem Fliessbett von Katalysatorpartikeln bei Temperaturen unterhalb 260°C beschrieben, bei den der zu chlorierende Stoff, Sauerstoff und elementares Chlor, Chlorwasserstoff oder eine Mischung aus den beiden letztgenannten Stoffen, eingesetzt wird. Sofern in der genannten Schrift von Ethylen die Rede ist, wird aber jeweils nur Chlorwasserstoff und Sauerstoff ohne elementares Chlor verwendet. Eine Mischung von Chlor und Chlorwasserstoff wird beispielsweise für die Chlorierung von Methan, also einem gesättigten Kohlenwasserstoff, bei dem die Substitutionsreaktion des Chlors erwünscht ist, empfohlen. Es ist ferner aus DE-AS Nr. 1229999 bekannt, 1,2-Dichlorethan dadurch herzustellen, dass man in einer ersten Stufe überschüssiges Ethylen, Chlorwasserstoff und überschüssigen Sauerstoff in Form von Luft in Gegenwart eines bekannten Oxichlorierungskatalysators bei 180 bis 350°C umsetzt, die Restgase aus dieser Stufe nach Auswaschen des nicht umgesetzten Chlorwasserstoffs und der dadurch bedingten Kondensation eines Grossteils des entstandenen 1,2-Dichlorethans in einer zweiten Stufe mit Chlor in Gegenwart eines eisenhaltigen Katalysators bei 80 bis 250°C umsetzt. Weiterhin ist aus DE-OS Nr. 2649533 ein Verfahren zur Herstellung von 1,2-Dichlorethan durch Chlorierung von ethylenhaltigen Gasen in der Gasphase bekannt, wobei man die Umsetzung in Gegenwart von Kupfer(II)-chlorid und/oder Eisen(III)chlorid auf einem Träger als Katalysator bei Temperaturen von 80 bis 250°C in der Katalysatorschicht durchführt. Vorzugsweise verwendet man für dieses Verfahren Gase, die höchstens 10% Ethylen enthalten, bzw. ethylenhaltige Gase, die als Restgase aus einem Oxichlorierungsverfahren stammen. Beide letztgenannten Veröffentlichungen befassen sich mit Zweistufenverfahren, die einen beträchtlichen apparativen Aufwand erfordern.

Schliesslich wird in der DE-OS Nr. 2106016 ein Verfahren zur Herstellung von 1,2-Dichlorethan durch Umsetzen mit Ethylen, Sauerstoff und Chlorwasserstoff oder Chlor oder einem Gemisch aus Chlorwasserstoff und Chlor als Chlorierungsmittel in einem aus oxichlorierenden Katalysatorteilchen bestehenden Wirbelbett beansprucht, wobei die Wirbelbettdichte so eingestellt wird, dass die in Längsrichtung des Bettes zwischen einer Stelle, die etwa 12,7 cm oberhalb des Einsatzpunktes der Reaktion liegt und dem oberen Ende des Bettes über dessen Breite, einem Temperaturgefälle von 11,1 bis 127,8°C entspricht. Über die Oxichlorierung des Ethylens mit Sauerstoff und Gemischen aus Chlor und Chlorwasserstoff werden jedoch in der Beschreibung und den Beispielen keinerlei Angaben gemacht.

Es wurde nun ein Verfahren gefunden, das es ermöglicht, 1,2-Dichlorethan in einem Reaktionsraum, der ein Katalysatorfestbett enthält, in einer Stufe durch Chlorierung und Oxichlorierung von Ethylen herzustellen, wobei im Hinblick auf die spätere thermische Zersetzung des 1,2-Dichlorethans zu Vinylchlorid günstige Molverhältnisse der Chlorierungsmittel eingesetzt werden können.

Es handelt sich um ein Verfahren zur Herstellung von 1,2-Dichlorethan durch Umsetzung von Ethylen mit Chlor Chlorwasserstoff und Sauerstoff oder Sauerstoff enthaltenden Inertgasen bei 180 bis 260°C und einem Druck von 0,09 bis 1,1 MPa in Gegenwart eines Kupfer(chlorid) enthaltenden Festbettkatalysators in einem Reaktionsraum, der heiz- und kühlbare Flächen enthält, wobei das gasförmige Reaktionsgemisch nach Durchströmen des Festbettkatalysators aus dem Reaktionsraum abgeführt, gekühlt und nach bekannten Verfahren das gebildete 1,2-Dichlorethan abgetrennt wird, mit dem Kennzeichen, dass in den Reaktionsraum vor Beginn des Katalysatorfestbettes Ethylen, Chlor sowie Sauerstoff oder Sauerstoff enthaltende Inertgase und entweder an der gleichen Stelle wie die vorgenannten Gase oder im ersten Drittel des Katalysatorfestbettes Chlorwasserstoff in folgenden Mengenverhältnissen eingeleitet werden: 1 mol $C_2H_4$: 0,35 bis 0,7 mol $Cl_2$: 0,7 bis 1,4 mol HCl: 0,2 bis 0,6 mol $O_2$.

Unter Inertgasen sind solche Stoffe zu verstehen, die unter den Reaktionsbedingungen gasförmig sind und an der Reaktion nicht oder nur in ganz untergeordnetem Masse teilnehmen. Beispiele hierfür sind Stickstoff, Kohlendioxid und Edelgase. Vorzugsweise wird Stickstoff in Mischung mit Sauerstoff eingesetzt, insbesondere atmosphärische Luft und Gemische von Luft und Sauerstoff.

Die Umsetzung wird bei einer Höchsttemperatur von 180 bis 260° C durchgeführt. Im allgemeinen wird die höchste Temperatur im Reaktionsraum im in Gasströmungsrichtung ersten Viertel des Katalysatorfestbettes gemessen. Diese Temperatur kann praktisch im gesamten Katalysatorfestbett herrschen, häufig wird jedoch ein Temperaturgefälle in Gasströmungsrichtung beobachtet, so dass am Ende der Katalysatorfestbettes eine um etwa 5 bis 50° C niedrigere Temperatur gemessen wird als an der Stelle der Maximaltemperatur.

Unterhalb 180° C werden ungünstige Ausbeuten beobachtet. Oberhalb 260° C nehmen die Verunreinigungen und Nebenprodukte erheblich zu, was wiederum die Ausbeute verschlechtert und erhöhten Aufwand zur Reindarstellung des 1,2-Dichlorethans erfordert. Vorzugsweise wird im Temperaturbereich von 190 bis 240° C und insbesondere von 200 bis 230° C gearbeitet.

Der Druck im Reaktionsraum beträgt zweckmässig 0,09 bis 1,1 MPa. Unterhalb normalem Atmosphärendruck, das heisst unterhalb 0,09 MPa sind erhöhte Apparatekosten notwendig bei sinkender Raum-Zeit-Ausbeute. Oberhalb 1,1 MPa nimmt zwar die Raum-Zeit-Ausbeute weiterhin zu, dieser Vorteil wird jedoch durch zusätzliche Apparateaufwendungen überkompensiert. Vorteilhaft wird im Druckbereich von 0,3 bis 0,9 MPa gearbeitet.

Der Reaktionsraum ist vorteilhaft überwiegend, das heisst zu 75% und mehr mit dem Festbettkatalysator gefüllt. Im Reaktionsraum, und zweckmässig insbesondere im Festbettkatalysator, der im allgemeinen aus einer Aufschüttung von Katalysatorteilchen besteht, sind heiz- und kühlbare Flächen angebracht. In der Regel wird das Reaktionsgefäss, in dem sich der Reaktionsraum befindet, einen Doppelmantel enthalten. Sofern der Reaktionsraum sehr schlank ist, beispielsweise ein Verhältnis von seiner Querschnittsfläche zu seiner Länge von 0,008 m²/m oder weniger aufweist, genügt dies. Ist das Verhältnis von Querschnittsfläche zu Länge deutlich grösser, werden zweckmässig Einbauten verwendet, als solche sind beispielsweise senkrecht stehende Platten oder Rohre geeignet. Der Doppelmantel bzw. die Rohre oder Platten werden von einem Temperiermedium durchflossen, wobei — beispielsweise wenn senkrechte Rohre als Einbauten verwendet werden — sich sowohl das Temperiermedium in den Rohren und der Katalysator ausserhalb sowie auch umgekehrt der Katalysator in den Rohren und das Temperiermedium ausserhalb befinden kann. In letzterem Fall entfällt der Doppelmantel.

Als Temperiermedium werden Flüssigkeite und/oder Gase verwendet, die zweckmässig so eingesetzt werden, dass eine gute Ausnutzung der abgeführten Reaktionswärme möglich ist. Als Flüssigkeiten seien beispielsweise genannt, hochtemperaturbeständige Öle, insbesondere Siliconöle oder gegebenenfalls verdampfendes Wasser oder 1,2-Dichlorethan. Als gasförmige Stoffe kommen beispielsweise Luft oder Ethylen in Frage, die nach ihrer Erwärmung im Reaktionsprozess eingesetzt werden oder auch Wasser- bzw. 1,2-Dichlorethandampf.

In den Reaktionsraum wird vor Beginn des Katalysatorfestbettes Ethylen, Chlor sowie Sauerstoff oder Sauerstoff enthaltendes Inertgas eingeleitet. Hierbei werden zweckmässig Ethylen und Chlor getrennt voneinander eingeführt, dagegen können Sauerstoff oder Sauerstoff enthaltende Inertgase zusammen mit dem Chlor eingeführt werden. In jedem Fall ist im Reaktionsraum für eine gute Durchmischung der Gase zu sorgen. Dies kann beispielsweise dadurch geschehen, dass das jeweilige Ende eines Einleitungsrohres mit einer Vorrichtung zur besseren Verteilung der Gase über die Fläche des Reaktionsraums, beispielsweise einer Fritte oder gelochten Platte, versehen ist. Ferner kann es vorteilhaft sein, unterhalb des Katalysatorfestbettes eine Aufschüttung von inerten Partikeln anzuordnen, die gleiche Grösse und Gestalt wie die katalysatorhaltigen Partikel haben können und gleichfalls dem Zweck dienen, die Gase gleichmässig zu verteilen.

Neben den oben beschriebenen Gasen wird noch Chlorwasserstoff, und zwar spätestens im in Gasströmungsrichtung ersten Drittel des Katalysatorfestbettes, eingeleitet. Die Chlorwasserstoffzugabe kann jedoch auch schon vorher, beispielsweise in der weiter oben beschriebenen Aufschüttung von Inertteilen oder zweckmässig zusammen mit dem Ethylen, durch eine Verteilervorrichtung erfolgen.

Die in den vorangegangenen Abschnitten genannten Gase werden in folgenden Mengenverhältnissen eingeleitet: 1 mol $C_2H_4$/0,35 bis 0,7 mol $Cl_2$/0,7 bis 1,4 mol HCl/0,2 bis 0,6 mol $O_2$. Diese Molverhältnisse sind besonders günstig im Hinblick auf die Weiterverwendung des erzeugten 1,2-Dichlorethans zur Herstellung von Vinylchlorid durch thermische Spaltung und Wiederverwendung des hierbei entstehenden Chlorwasserstoffs. Innerhalb dieser Molverhältnisse werden gute Ausbeuten an 1,2-Dichlorethan ohne grösseren Anfall von Nebenprodukten erhalten, insbesondere wenn auf 1 mol Ethylen nicht mehr als etwa 2 Atome Chlor (berechnet aus der Summe von Chlor und Chlorwasserstoff) angewendet werden. Vorzugsweise wird innerhalb folgender Molverhältnisse gearbeitet: 1 mol $C_2H_4$/0,40 bis 0,55 mol $Cl_2$/0,9 bis 1,2 mol HCl/0,25 bis 0,4 mol $O_2$. Wie weiter oben bereits ausgeführt, kann der Sauerstoff günstig in Form von Luft oder Gemischen von Luft und Sauerstoff in den Prozess eingebracht werden.

Es ist weiterhin von Vorteil, mit etwas mehr Sauerstoff, dass heisst etwa 125 bis 180 Vol.% der für die Umsetzung notwendigen stöchiometrischen Menge, zu arbeiten. Hierdurch wird die Ausbeute in bezug auf die anderen Gase verbessert und Schwierigkeiten in der Aufarbeitung des Reaktionsgemisches, die zu einem erhöhten apparativen Aufwand Anlass geben können, werden vermieden, insbesondere wenn Luft als Sauerstoffquelle eingesetzt wird.

Vorteilhaft ist es ferner, einen Überschuss von Ethylen in bezug auf die Chlor enthaltenden Gase einzusetzen, dergestalt, dass auf 1 mol Ethylen 1,8 bis 1,99 Atome Cl, berechnet aus der Summe von $Cl_2$ und HCl, eingesetzt werden. Hierdurch werden Schwierigkeiten durch Korrosion und Nebenreaktionen vermieden.

Das Katalysatorfestbett besteht zweckmässig aus einer Aufschüttung von Teilchen der Grösse von 0,1 bis 15, vorzugsweise 1 bis 7 mm. Die einzelnen Teilchen können unterschiedliche Gestalt aufweisen. Sie können beispielsweise kugel-, würfel-, quader- oder zylinderförmig sein sowie die Form von Stäbchen, Halbkugeln oder Ringen aufweisen.

Der Katalysator kann aus Teilchen gleicher Zusammensetzung bestehen, vorzugsweise besteht er jedoch aus Teilchen verschiedener Zusammensetzung. Hierbei brauchen nicht alle Teilchen Kupfer zu enthalten, es ist im Gegenteil insbesondere bei Festbetten in grösseren Behältern, die ein relativ hohes Verhältnis von Volumen zu Oberfläche aufweisen, vorteilhaft, den kupferhaltigen Katalysatorteilchen inerte Teilchen zuzusetzen, die aus einem Stoff bestehen, der bei 200°C eine Wärmeleitzahl von mindestens 400 $Jh^{-1}$ $cm^{-1}$ $K^{-1}$ aufweist. Geeignete Stoffe hierfür sind beispielsweise Graphit oder Metalle oder Metalllegierungen, die mit dem Gasgemisch unter den Reaktionsbedingungen nicht oder nur in untergeordnetem Masse reagieren wie Nickel, Silber, Nickellegierungen oder nickellegierte Stähle mit relativ hohem Nickelgehalt. Günstige Ergebnisse werden erhalten wenn 25 bis 75 Vol.%, bezogen auf das Volumen aller Teilchen des Festbettes, aus den beschriebenen inerten Teilchen bestehen. Unter 25 Vol.% wird im allgemeinen keine deutlich verbesserte Wärmeableitung festgestellt, oberhalb 75 Vol.% lässt die Aktivität des Katalysators, offenbar wegen zu grosser Verdünnung, merklich nach.

Die Teilchen des Katalysatorfestbettes, die den eigentlichen Katalysator enthalten, bestehen vorzugsweise aus porösem Aluminiumoxid oder Siliciumdioxid oder Mischungen dieser beiden Oxide, auf denen das Kupfer im allgemeinen als eine Kupferverbindung, meistens als ein Kupferchlorid niedergeschlagen ist. Die Katalysatorteilchen können auch andere Kupfersalze, beispielsweise das Nitrat oder Acetat, sowie andere Kupferverbindungen enthalten, während der Benutzung des Katalysators werden diese jedoch in der Regel in Kupferchloride umgewandelt.

Es ist von Vorteil, ein Katalysatorfestbett zu verwenden, das in Strömungsrichtung der Gase entweder kontinuierlich oder in Stufen steigende Kupferkonzentration enthält. Hierdurch wird unter anderem eine bessere Wärmeverteilung über den gesamten Katalysator und dadurch auch eine bessere Abführung der Reaktionswärme erreicht. Diese Konzentrationszunahme in Strömungsrichtung der Gase sollte so eingestellt werden, dass das Katalysatorfestbett beim Eintritt der Gase mindestens 1, vorzugsweise 3 bis 8 Gew.%, Kupfer enthält und beim Austritt der Gase höchstens 20, vorzugsweise 4 bis 12 Gew.% Kupfer. Unterhalb 1 Gew.% Kupfer lässt die katalytische Wirkung zu stark nach, oberhalb 20 Gew.% Kupfer ist der Katalysator im allgemeinen nicht mehr ausreichend porös, um eine wünschenswert grosse Oberfläche mit dem Reaktionsgemisch in Kontakt zu bringen, ausserdem steigt die Gefahr, dass Kupferverbindungen flüchtig mit dem Reaktionsgemisch aus dem Reaktionsraum ausgetragen werden. Im Fall der Mitverwendung von Teilchen aus inertem, gut wärmeleitfähigem Material, wie oben beschrieben, muss die Kupferkonzentration der katalysatorhaltigen Teilchen entsprechend höher gehalten werden, um die obengenannten, auf das gesamte Festbett bezogenen Werte zu erreichen, jedoch sollte auch in diesem Falle die Kupferkonzentration der katalysatorhaltigen Teilchen 20 Gew.% nicht überschreiten.

Neben Kupfer enthalten die Katalysatorteilchen vorteilhaft wenigstens eine der folgenden Verbindungen: Natrium-, Kalium-, Calcium- oder Magnesiumchlorid. Hierdurch wird die Wirksamkeit des Katalysators verbessert bzw. die Flüchtigkeit der Kupferverbindungen herabgesetzt. Die Menge der zugesetzten obengenannten Verbindung richtet sich nach dem Kupfergehalt und beträgt zweckmässig etwa 25 bis 120 Gew.%, bezogen auf den Kupferanteil des Katalysators.

Ferner kann die Wirksamkeit der Katalysatorteilchen gesteigert werden durch Zusatz von 0,001 bis 1 Gew.%, bezogen auf das Gesamtgewicht des Festbettes an Eisen, das auch in Form einer Verbindung vorliegen kann, die unter den Reaktionsbedingungen in Eisenchlorid verwandelt wird. Anstelle von Eisenchlorid kann auch eine andere Lewis-Säure verwendet werden.

Neben den genannten Zusätzen kann der Katalysator noch weitere Zusätze enthalten, die die Wirksamkeit und/oder Selektivität des Katalysators in bezug auf die Gewinnung von 1,2-Dichlorethan verbessern. Als Beispiele seien genannt Silber, Zink, Chrom, Mangan, seltene Erden wie Cer, Lanthan, Ytterbium, Neodym und Praseodym; Platinmetalle, Rhodium, Platin.

Das Katalysatorfestbett sollte zweckmässig einen freien Gasraum von 15 bis 60, vorzugsweise 20 bis 45 Vol.% besitzen. Unterhalb 15 Vol.% wird der Druckabfall im Katalysatorfestbett im allgemeinen zu hoch, so dass nicht die für eine wirtschaftliche Arbeitsweise notwendigen Gasmengen durchgesetzt werden können. Ein Katalysatorfestbett mit einem freien Gasraum von über 60 Vol.% erfordert eine komplizierte Gestalt der einzelnen Katalysatorteilchen, macht den Katalysator mechanisch wenig stabil und erfordert einen grösseren Reaktionsraum und damit erhöhte Investitionskosten. Auch wird die Wärmeableitung innerhalb des Katalysators verschlechtert. Der freie Gasraum eines Katalysatorfestbettes kann beispielsweise wie folgt bestimmt werden: In ein zylindrisches Messgefäss werden genau 2 l Katalysatorteilchen eingefüllt und Gefäss und Füllung gewogen. Nun wird so lange Wasser zugesetzt, bis der Wasserspiegel die Oberfläche der Katalysatorteilchen-Aufschüttung erreicht hat. Das zylindrische Gefäss wird hierbei ab und zu leicht geschüttelt, um eingeschlossene Luft entweichen zu lassen. Nun wird wiederum das Gefäss mit dem Inhalt gewogen. Die Gewichtsdifferenz in Gramm ist praktisch gleich dem freien Gasraum der Katalysatorteilchen-Aufschüttung in Kubikzentimeter. Der Reaktionsraum ist zweckmässig zu 75 bis 98 Vol.%, vorteilhaft zu 85 bis 95 Vol.%, mit dem Katalysatorfestbett gefüllt. Die mittlere Verweilzeit der eingeführten Gase im Reaktionsraum beträgt im allgemeinen 4 bis 50 s, vorzugsweise 7 bis 25 s. Unter 4 s mittlerer Verweilzeit wird ein schlechter Umsatz beobachtet, oberhalb 50 s ist das Verfahren zwar durchführbar, aber mit deutlich schlechterer Raum-Zeit-Ausbeute und damit schlechterer Wirtschaftlichkeit.

Nach Verlassen des Katalysatorfestbettes wird die Reaktionsmischung wie üblich gekühlt und durch partielle Kondensation bzw. Destillation das 1,2-Dichlorethan von den nicht umgesetzten Reaktionspartnern sowie von Inertgasen und Nebenprodukten befreit.

Wie bereits weiter oben ausgeführt, ermöglicht es das erfindungsgemässe Verfahren Ethylen in einem einzigen Reaktionsraum in Gegenwart eines Festbettkatalysators mit Chlor, Chlorwasserstoff und Sauerstoff in Mengenverhältnissen umzusetzen, die besonders günstig sind im Hinblick auf die spätere thermische Zersetzung von 1,2-Dichlorethan zur Gewinnung von Vinylchlorid. Das 1,2-Dichlorethan wird mit guten Ausbeuten ohne wesentliche Mengen von störenden Nebenprodukten erhalten. Der Umsatz in bezug auf Chlor bzw. Chlorwasserstoff ist ebenfalls gut. Das in der Reaktionsmischung vorhandene Chlor wird vollständig in 1,2-Dichlorethan verwandelt. Das neue Verfahren ist apparativ wenig aufwendig und erzeugt die Reaktionswärme bei der Chlorierung des Ethylens auf einem Temperaturniveau, das es gestattet, die Energie günstig wiederzuverwenden, beispielsweise zur Heizung von Destillationsapparaturen.

Nachfolgende Beispiele sollen das neue Verfahren näher erläutern.

*Beispiel 1*

Es wird folgende Apparatur verwendet:

Zur Ausführung der Umsetzung von Ethylen zu 1,2-Dichlorethan dient ein senkrecht stehendes Glasrohr mit 50 mm innerem Durchmesser, das unten und oben zu je einer Gaseinström- bzw. Gasausströmöffnung verjüngt ist. Dieses senkrecht stehende Reaktionsrohr enthält unmittelbar über der unteren Einströmöffnung eine Glasfritte, die über den gesamten inneren Querschnitt des Reaktionsrohres verläuft. In kurzem Abstand über dieser ersten Fritte ist eine zweite Fritte angebracht, deren Fläche etwa den halben Querschnitt des Reaktionsrohres ausmacht und die in ihrem unteren Teil mit einem Glasrohr verbunden ist, das seitlich durch den Mantel des Reaktionsrohres geführt ist. Zur Temperierung enthält das Reaktionsrohr eine Glasrohrschlange, deren Anschlüsse ebenfalls seitlich durch den Mantel des Reaktionsrohres geführt sind und die kurz oberhalb der zweiten Fritte beginnt und im Reaktionsrohr so weit nach oben reicht, dass etwa 5/100 der gesamten Länge des Reaktionsrohres im oberen Teil freibleibt. Im Reaktionsrohr sind in gleichen Abständen voneinander und vom Boden und Kopf des Reaktionsrohres entfernt zwei Zwischenböden angebracht, die aus gelochten Platten bestehen. Ausserdem trägt der Mantel des Reaktionsrohres 4 Stutzen, von denen 3 je etwa in der Mitte der Rohrteile angebracht sind, die durch die beiden Zwischenböden voneinander abgeteilt werden. Ein vierter Stutzen befindet sich kurz unterhalb des ersten Zwischenbodens etwas unterhalb des in Gasströmungsrichtung ersten Drittels des Reaktionsrohres. Ferner enthält das Reaktionsrohr vier Temperaturmessstellen ($T_1$-$T_4$), die gleichfalls durch den Rohrmantel geführt sind. Zwei dieser Messstellen ($T_1$, $T_2$) befinden sich unterhalb des in Gasströmungsrichtung ersten Zwischenbodens. Die erste Messstelle ($T_1$) im in Gasströmungsrichtung ersten Viertel des gesamten Reaktionsrohres, die zweite Messstelle ($T_2$) kurz unterhalb des ersten Zwischenbodens. Die weiteren Temperaturmessstellen ($T_3$, $T_4$) sind jeweils in der Mitte des in Gasströmungsrichtung zweiten ($T_3$) und dritten Drittels ($T_4$) des Reaktionsrohres angebracht.

Das Reaktionsrohr enthält im untersten Drittel einen Doppelmantel, durch den Gase oder Flüssigkeiten geleitet werden können, im mittleren und oberen Drittel ist der Mantel wärmeisoliert. Die Länge des Reaktionsraumes, gemessen von der Oberfläche der ersten Fritte bis zur Verjüngungsstelle im obersten Teil des Reaktionsrohres, beträgt 750 mm.

Das Gasableitungsrohr vom Oberteil des Reaktionsrohres ist über eine absteigende Leitung mit einem Wasserkühler verbunden, an dessen unterem Ende ein Kondensatsammelgefäss mit Ablasshahn angebracht ist. Das Kondensatsammelgefäss enthält in seinem Oberteil ein Gasabführungsrohr, das wiederum in einen aufsteigenden Solekühler mündet. Die hier kondensierten Bestandteile des Gases fliessen in ein zweites Kondensatsammelgefäss mit Ablasshahn. Die den oberen Teil des Solekühlers verlassenden, nicht kondensierbaren Abgase werden durch Waschflaschen geleitet, zur Abscheidung des darin enthaltenen Chlorwasserstoffs. Von dem gewaschenen Abgas werden Proben für eine gaschromatographische Analyse entnommen. Die Kondensate, die sich in den beiden unterhalb der Kühler angebrachten Gefässe sammeln, werden vereinigt und ebenfalls gaschromatographisch analysiert. Das Überleitungsrohr vom Reaktionsrohr bis zu ersten Kühler ist mit einer Heizmanschette versehen und wird während des Betriebes des Reaktors soweit geheizt, dass hier keine Kondensatbildung auftritt.

Das Volumen des Reaktionsraumes im Reaktionsrohr, abzüglich der darin enthaltenen Einbauten (Temperierschlange, zweite Fritte sowie Temperaturmessfühler), beträgt 1150 cm³. Zur Durchführung des ersten Beispiels wird das Reaktionsrohr mit einem Katalysator gefüllt, der auf Alumiumoxid Kupferchlorid in verschiedener Konzentration enthält und aus zylindrischen Einzelteilchen besteht, deren Boden und Deckfläche je 4,3 mm Durchmesser und deren Mantellinie 4,3 mm Länge aufweist. Das untere Drittel des Reaktionsrohres bis zum ersten Zwischenboden wird mit Katalysatorteilchen gefüllt, die 3,7 Gew.% Kupfer, ca. 3,3 Gew.% Kaliumchlorid und ca. 0,005 Gew.% Eisen enthalten, wobei alle Gewichtsprozentangaben sich auf das Gesamtgewicht der Katalysatorteilchen beziehen. Das mittlere Drittel des Reaktionsrohres, zwischen den beiden Zwischenböden, wird mit einem Katalysator gefüllt, der 5,1 Gew.% Kupfer, ca. 3,3 Gew.% Kaliumchlorid und ca. 0,005 Gew.% Eisen enthält. Das oberste Drittel wird mit Katalysatorteilchen der Zusammensetzung 7,8 Gew.% Kupfer, 3,3 Gew.% Kaliumchlorid und ca. 0,005 Gew.% Eisen gefüllt. Für jedes Rohrdrittel werden 0,35 l Katalysatorteilchen verwendet, so dass insgesamt 1,05 l Katalysatorteilchen im Reaktionsrohr aufgeschüttet sind, das sind 91,3 Vol.% des gesamten zur Verfügung stehenden Reaktionsraumes. Das freie Gasvolumen der aufgeschütteten Katalysatorteilchen wird, wie oben beschrieben, nach der Wasserverdrängungsmethode zu 35. Vol.% bestimmt.

Durch die Glasrohrschlange wird zunächst heisses Öl geleitet und damit der Katalysator auf ca. 190°C erwärmt. Nun wird über das untere Gaseinleitungsrohr durch die erste Fritte Luft in einer Menge von 40 Normalliter/h und Chlor in einer Menge von 10 Normalliter/h in das Reaktionsrohr eingeleitet. Gleichzeitig wird über die zweite Fritte das Reaktionsrohr mit einer Mischung aus 22 Normalliter/h Ethylen und 24 Normalliter/h Chlorwasserstoffgas beschickt. Das Molverhältnis der eingeleiteten Gase beträgt: 1 mol $C_2H_4$/0,435 mol $Cl_2$/1,044 mol HCl/0,365 mol $O_2$ (in Form von Luft). Bezogen auf die zur Oxidation des Chlorwasserstoffs notwendige stöchiometrische Menge (= 100 Vol.%) beträgt der Sauerstoffüberschuss 139,4 Vol.%. Die Chlor- und Chlorwasserstoffmenge ist so bemessen, dass auf 1 mol Ethylen 1,9 Atome Chlor kommen.

Zusammen mit der Einleitung der Reaktionsgase wird der Wasserkühler mit Wasser von 14°C und der Solekühler mit Kühlsole von −15°C beschickt. Die Abgaswaschflaschen enthalten Wasser als Waschflüssigkeit. Nach kurzer Zeit wird am im ersten Viertel des Reaktionsrohres befindlichen Temperaturmessfühler eine Temperatursteigerung auf ca. 230°C festgestellt. Bereits während dieser Zeit wurde die Temperatur des durch die Glasrohrschlange fliessenden Öles laufend gesenkt und nach ca. ½ h so einreguliert, dass an den vier Temperaturmessstellen $T_1$ bis $T_4$ in Gasströmungsrichtung folgende Temperaturen abgelesen werden, die innerhalb der nächsten 3 h konstant bleiben:
$T_1$ = 243°C, $T_2$ = 217°C, $T_3$ = 215°C, $T_4$ = 212°C.

Zusätzlich wird zur Konstanthaltung der Temperatur im unteren Drittel des Reaktionsrohres bei Bedarf Kühlluft durch den Doppelmantel geblasen. Das den Solekühler verlassende Abgas hat eine Temperatur von +12°C.

Der Versuch wird während 3 h fortgeführt und nach 1/3 und 2/3 dieser Zeit je einmal die Abgaszusammensetzung des gewaschenen Abgases gaschromatographisch ermittelt. Für die gase Sauerstoff, Kohlenoxid, Kohlendioxid und Ethylen wird ein Wärmeleitfähigkeits-Detektor, für alle anderen nachstehend angegebenen Gase ein Flammionisationsdetektor verwendet. Die Mittelwerte aus beiden Analysen sind für alle Beispiele in der weiter unten stehenden Tabelle II zusammengefasst, wobei vom Sauerstoffwert der über die verwendete Luft mitgebrachte Edelgasteil bereits abgerechnet ist.

Nach Ablauf der Versuchsdauer wird die Gaszufuhr zum Reaktionsrohr beendet und der Katalysator mit Luft von Zimmertemperatur kaltgeblasen. Das vom Wasser und Solekühler gebildete Kondensat wird vereinigt, gewogen und ebenfalls gaschromatographisch mit einem Flammionisationsdetektor analysiert. Die für die einzelnen Beispiele ermittelten Werte sind in der weiter unten stehenden Tabelle I aufgeführt.

Für den Versuch gemäss Beispiel 1 werden folgende Werte errechnet:
Umsatz, bezogen auf: HCl = 86%; $Cl_2$ = 100%; $C_2H_4$ = 91%. Raum-Zeit-Ausbeute (bezogen auf 0,45 l Reaktionsraum): 221,25 g Roh-1,2-dichlorethan · h⁻¹ · l⁻¹.

Mittlere Verweilzeit der Gase im Reaktionsraum: 8,0 s.

### Beispiel 2

Es wird dieselbe Apparatur verwendet wie im Beispiel 1 und genauso verfahren wie dort angegeben mit dem Unterschied, dass die Temperatur im untersten Viertel des Katalysatorfestbettes im Reaktionsrohr auf 220°C während einer Versuchsdauer von insgesamt 3,5 h gehalten wird.

Folgende Werte werden ermittelt: Molverhältnis 1 mol $C_2H_4$/0,435 mol $Cl_2$/1,044 mol HCl/0,365

mol $O_2$ (als Luft). $O_2$-Überschuss sowie Verhältnis Ethylen zu Chlor wie in Beispiel 1.

Temperaturen: $T_1 = 220°C$, $T_2 = 217°C$, $T_3 = 214°C$, $T_4 = 198°C$.

Umsatz, bezogen auf: $HCl = 98\%$; $Cl_2 = 100\%$; $C_2H_4 = 98,5\%$. Raum-Zeit-Ausbeute (bezogen auf 0,45 l Reaktionsraum): 239,5 g Roh-1,2-dichlorethan $\cdot$ $h^{-1} \cdot l^{-1}$.

Mittlere Verweilzeit der Gase im Reaktionsraum: 7,9 s. Analysen: s. Tabellen I und II.

### Beispiel 3

Wiederum wird verfahren wie im Beispiel 1 bzw. Beispiel 2, jedoch mit dem Unterschied, dass der Chlorwasserstoff nicht über die zweite Fritte in den Reaktionsraum eingeleitet wird, sondern über ein Rohr mit kugelförmigem gelochtem Abschluss, das durch den seitlich am Mantel des Reaktionsrohres angebrachten Stutzen, der sich kurz unterhalb des ersten Zwischenbodens befindet, in das Reaktionsrohr eingeführt ist.

Die im ersten Viertel des Katalysatorfestbettes gemessene Temperatur wird auf 235°C gehalten. Die Versuchsdauer beträgt 3 ¾ h.

Folgende Werte werden ermittelt: Temperaturen: $T_1 = 235°C$, $T_2 = 228°C$, $T_3 = 219°C$, $T_4 = 217°C$.

Umsatz, bezogen auf: $HCl = 85\%$; $Cl_2 = 100\%$; $C_2H_4 = 91\%$. Raum-Zeit-Ausbeute (bezogen auf 0,45 l Reaktionsraum): 221,5 g Roh-1,2-dichlorethan $\cdot$ $h^{-1} \cdot l^{-1}$.

Mittlere Verweilzeit der Gase im Reaktionsraum: 7,8 s.

Analysen: s. Tabellen I und II.

### Beispiel 4

Es wird die gleiche Apparatur verwendet wie in Beispiel 1, jedoch werden in allen drei Abteilungen des Reaktionsrohres die gleichen Katalysatorteilchen eingefüllt, es handelt sich um zylindrische Teilchen mit einem Durchmesser der Deck- und Bodenfläche von 4,3 mm und einer Länge der Mantellinie von 4,3 mm. Diese Teilchen bestehen aus Aluminiumoxid mit 7,8 Gew.% Kupfer (als Kupferchlorid) und 3,3 Gew.% Kaliumchlorid sowie 0,005 Gew.% Eisen, wobei sich alle Gewichtsprozentangaben auf das Gesamtgewicht der Katalysatorteilchen beziehen. Es werden insgesamt 1,05 l Katalysatorteilchen eingefüllt. Die Katalysatoraufschüttung nimmt 91,3 Vol.% des Reaktionsrohres ein. Im übrigen wird verfahren wie in Beispiel 1 beschrieben, wobei die Temperatur im ersten Viertel des Katalysatorfestbettes auf 241°C eingestellt wird. Die Versuchsdauer beträgt 2,5 h.

Folgende Werte werden ermittelt:

Temperatur: $T_1 = 241°C$, $T_2 = 216°C$, $T_3 = 212°C$, $T_4 = 199°C$.

Umsatz, bezogen auf: $HCl = 89\%$; $Cl_2 = 100\%$; $C_2H_4 = 94\%$. Raum-Zeit-Ausbeute (bezogen auf 0,45 l Reaktionsraum): 228,75 g Roh-1,2-dichlorethan $\cdot$ $h^{-1} \cdot l^{-1}$.

Mittlere Verwelzeit der Gase im Reaktionsraum: 8,1 s.

Analysen: s. Tabellen I und II.

### Beispiel 5

Die hierfür verwendete Apparatur ist analog der im Beispiel 1 verwendeten aufgebaut, jedoch mit dem Unterschied, dass als Reaktionsraum ein senkrecht stehendes Nickelrohr mit 50 mm innerem Durchmesser verwendet wird, das ähnlich dem Glasrohr der im Beispiel 1 verwendeten Apparatur ausgestattet ist mit folgendem Unterschied: Es sind nur drei Temperaturmessstellen vorhanden, von denen die erste ($T_1$) im in Gasströmungsrichtungsrichtung ersten Viertel des Katalysatorbettes, die zweite ($T_2$) in der Mitte und die dritte ($T_3$) im letzten Viertel des Katalysatorfestbettes angebracht ist. Die gelochten Zwischenböden entfallen. Im Gasableitungsrohr am Kopf des Reaktors ist ein Druckminderventil mit einer Druckregelung angebracht. Der freie Reaktionsraum im Rohr beträgt 1,5 l. In diesen werden nacheinander in gleichen Portionen zu je 0,45 l zylindrische Katalysatorteilchen eingefüllt, die eine Deck- und Bodenfläche mit 4,3 mm Durchmesser und eine Mantellinie mit 4,3 mm Länge aufweisen. Die Art der Katalysatorteilchen ist dieselbe wie im Beispiel 1 beschrieben, dass heisst die erste Portion enthält 3,7, die zweite Portion 5,1 und die dritte Portion 7,8 Gew.% Kupfer, bezogen auf das Gesamtgewicht der Katalysatorteilchen. Der für die Gasreaktion zur Verfügung stehende freie Raum beträgt 0,62 l.

Am Reaktorausgang, nach dem Druckminderventil, sind Wasser und Solekühler sowie Chlorwasserstoffwäscher angeschlossen, wie im Beispiel 1 beschrieben.

Vor Beginn des Versuches wird das Katalysatorfestbett im Reaktionsrohr vermittels der Nickelrohrschlange auf 190°C aufgeheizt. Nun werden durch die unterste Gaszuführungsleitung und Fritte 1 120 Normalliter/h Luft und 30 Normalliter/h Chlorgas unter Druck eingeleitet und durch Regulierung des Druckminderventils am Kopf des Reaktors ein Druck von 0,4 MPa im Reaktor eingestellt und während der gesamten Versuchsdauer gehalten. Gleichzeitig werden über die Fritte 2 63 Normalliter/h Ethylen und 60 Normalliter/h Chlorwasserstoffgas unter Druck in das Reaktionsrohr eingetragen. Das Molverhältnis der eingeführten Gase beträgt 1 mol $C_2H_4$/0,476 mol $Cl_2$/0,956 mol $HCl$/0,4 mol $O_2$ (als Luft). Der $O_2$-Überschuss über die stöchiometrisch zur Oxidation des Chlorwasserstoffs notwendige Menge beträgt 168 Vol.%. Je Molekül Ethylen werden 1,9 Atome Chlor eingetragen.

Während der gesamten Versuchsdauer von 5 h ist der Wasserkühler mit Wasser von 14°C und der Solekühler mit Kühlsole von −18°C durchflossen. Das Abgas, das den Solekühler verlässt, hat eine Temperatur von 12°C.

Etwa 25 min nach Beginn des Versuches hat sich im ersten Viertel des Katalysatorfestbettes eine Temperatur von 235°C eingestellt, die im weiteren Verlauf des Versuches durch Kühlung der Nickelrohrschlange, wie im Beispiel 1 beschrieben, gehalten wird. Folgender Temperaturverlauf wird gemessen: $T_1 = 235°C$; $T_2 = 225°C$; $T_3 = 218°C$.

Nach 1/3 und 2/3 der gesamten Versuchsdauer werden Abgasproben nach der Chlorwasserstoffwäsche gaschromatographisch untersucht. Der Mittelwert der festgestellten Ergebnisse ist in nachfolgender Tabelle II aufgeführt.

Nach Versuchsende werden die vom Wasser- bzw. Solekühler kondensierten Flüssigkeiten vereinigt, gewogen und anschliessend gaschromatographisch analysiert. Das Analysenergebnis ist in nachstehender Tabelle I aufgeführt.

Folgende Werte werden ermittelt:

Umsatz bezogen auf: HCl = 98%; $Cl_2$ = 100%; $C_2H_4$ = 99%. Raum-Zeit-Ausbeute (bezogen auf 0,62 l Reaktionsraum): 498 g Roh-1,2-dichlorethan $\cdot$ h$^{-1}$ $\cdot$ l$^{-1}$.

Mittlere Verweilzeit der Gase im Reaktionsraum: 9,8 s.

Die Beispiele 1 bis 4 werden bei normalem Atmosphärendruck durchgeführt.

*Beispiel 6*

Es wird dieselbe Apparatur verwendet wie im Beispiel 1 und genauso verfahren wie dort angegeben mit folgenden Unterschieden: Die Temperatur im untersten Viertel des Katalysatorfestbettes im Reaktionsrohr wird während einer Versuchsdauer von 2,75 h auf etwa 220°C gehalten. Die Festbettkatalysatorteilchen enthalten 11 Gew.% Kupfer, ca. 5,7 Gew.% Kaliumchlorid und 0,5 Gew.% Eisen. Das untere Drittel des Reaktionsrohres ist mit einer Mischung von 60 Gew.% der oben näher beschriebenen Katalysatorteilchen mit 40 Gew.% Glaskugeln von 4,5 mm Durchmesser, der Mittelteil des Reaktionsrohres mit 70 Gew.% Katalysatorteilchen und 30 Gew.% der beschriebenen Glaskugeln und das oberste Drittel des Reaktionsrohres mit 80 Gew.% Katalysatorteilchen und 20 Gew.% Glaskugeln gefüllt.

Nach Erwärmen des Katalysators wird über das untere Gaseinleitungsrohr durch die erste Fritte Luft in einer Menge von 40 Normalliter/h und Chlor in einer Menge von 10 Normalliter/h in das Reaktionsrohr eingeleitet. Gleichzeitig wird über die zweite Fritte das Reaktionsrohr mit einer Mischung aus 23 Normalliter/h Ethylen und 24 Normalliter/h Chlorwasserstoffgas beschickt. Das Molverhältnis der eingeleiteten Gase beträgt: 1 mol $C_2H_4$/0,45 mol $Cl_2$/1,1 mol HCl/0,38 mol $O_2$ (in Form von Luft). Bezogen auf die zur Oxidation des Chlorwasserstoffs notwendige stöchiometrische Menge (= 100 Vol.%) beträgt der Sauerstoffüberschuss 138 Vol.%. Die Chlor- und Chlorwasserstoffmenge ist so bemessen, dass auf 1 mol Ethylen 2 Atome Chlor kommen.

Folgende Werte werden ermittelt:

Temperaturen: $T_1$ = 219°C, $T_2$ = 220°C, $T_3$ = 224°C, $T_4$ = 202°C.

Umsatz bezogen auf: HCl = 98%; $Cl_2$ = 100%; $C_2H_4$ = 99%. Raum-Zeit-Ausbeute (bezogen auf 0,45 l Reaktionsraum): 240,7 g Roh-1,2-dichlorethan $\cdot$ h$^{-1}$ $\cdot$ l$^{-1}$.

Mittlere Verweilzeit der Gase im Reaktionsraum: 7,9 s.

Analysen: s. Tabelle I und II.

Tabelle I

Gaschromatographische Analyse des kondensierten Roh-1,2-dichlorethan

| Komponenten | Beispiel Nr. 1 (Gew.%) | Beispiel Nr. 2 (Gew.%) | Beispiel Nr. 3 (Gew.%) | Beispiel Nr. 4 (Gew.%) | Beispiel Nr. 5 (Gew.%) | Beispiel Nr. 6 (Gew.%) | Beispiel Nr. 7 (Gew.%) |
|---|---|---|---|---|---|---|---|
| 1,2 Dichlorethan | 98,91 | 99,26 | 98,78 | 99,03 | 98,06 | 99,22 | 99,12 |
| Summe: $C_2H_2$, $C_2H_4$, $C_2H_6$ | 0,0284 | 0,0273 | 0,0184 | 0,028 | 0,0053 | 0,0128 | 0,0413 |
| Vinylchlorid | 0,0046 | 0,0049 | 0,0041 | 0,0055 | 0,0014 | 0,0024 | 0,0057 |
| $C_2H_5Cl$ | 0,0429 | 0,0282 | 0,0295 | 0,0406 | 0,0093 | 0,0475 | 0,0319 |
| 1,1-Dichlorethylen | 0,0074 | 0,0012 | 0,0019 | | 0,0004 | | 0,0005 |
| 1,2-Dichlorethylen-trans | 0,0014 | 0,0042 | 0,0022 | 0,004 | 0,0087 | 0,001 | 0,0008 |
| 1,1-Dichlorethan | 0,0047 | 0,0039 | 0,0054 | 0,0031 | 0,0053 | 0,0033 | 0,004 |
| $CCl_4$ | 0,1041 | 0,0730 | 0,0897 | 0,163 | 0,0836 | 0,1266 | 0,1938 |
| 1,2-Dichlorethylen-cis | 0,0084 | 0,0108 | 0,0046 | 0,0233 | 0,0446 | 0,007 | 0,0059 |
| $CHCl_3$ | 0,0543 | 0,0453 | 0,0420 | 0,0857 | 0,0728 | 0,0238 | 0,0435 |
| 1,1,2-Trichlorethylen | 0,0039 | 0,0053 | 0,0017 | 0,0076 | 0,0101 | 0,0024 | 0,0025 |
| 1,1,2-Trichlorethan | 0,2820 | 0,2485 | 0,5894 | 0,433 | 1,0795 | 0,1948 | 0,1481 |
| 2-Chlorethanol | 0,0164 | 0,0155 | 0,0211 | 0,0081 | 0,0063 | 0,008 | 0,0076 |
| 1,1,2,2-Tetrachlorethan | 0,0047 | 0,0095 | 0,0350 | 0,0131 | 0,3011 | 0,0046 | 0,004 |
| Chloral | 0,5200 | 0,2530 | 0,3678 | 0,156 | 0,297 | 0,345 | 0,394 |

*Beispiel 7*

Es wird gearbeitet wie im Beispiel 6 beschrieben unter Verwendung des gleichen Katalysatorfestbettes. Nach Erwärmung des Katalysators wird über das untere Gaseinleitungsrohr durch die erste Fritte Luft in einer Menge von 40 Normalliter/h und Chlor in einer Menge von 14 Normalliter/h in das Reaktionsrohr eingeleitet. Gleichzeitig wird über die zweite Fritte das Reaktionsrohr mit einer Mischung aus 27 Normalliter/h Ethylen und 24 Normalliter/h Chlorwasserstoff beschickt. Das Molverhältnis der eingeleiteten Gase beträgt:

1 mol $C_2H_4$/0,54 mol $Cl_2$/0,92 mol $HCl$/0,32 mol $O_2$ (in Form von Luft). Bezogen auf die zur Oxidation des Chlorwasserstoffs notwendige stöchiometrische Menge (= 100 Vol.%) beträgt der Sauerstoffüberschuss 139 Vol.%. Die Chlor- und Chlorwasserstoffmenge ist so bemessen, dass auf 1 Molekül Ethylen 2 Atome Chlor kommen. Während einer Reaktionsdauer von 3,15 h werden folgende Werte ermittelt:

Temperaturen: $T_1$ = 218°C, $T_2$ = 222°C, $T_3$ = 223°C, $T_4$ = = 200°C.

Umsatz, bezogen auf: $HCl$ = 95%; $Cl_2$ = 100%; $C_2H_4$ = 91%. Raum-Zeit-Ausbeute (bezogen auf 0,45 l Reaktionsraum): 282 g Roh-1,2-dichlorethan · $h^{-1}$ · $l^{-1}$.

Mittlere Verweilzeit der Gase im Reaktionsraum: 7,5 S.

Analysen: s. Tabelle I und II.

### Tabelle II

Gaschromatographische Analyse des Abgases nach der Chlorwasserstoffwäsche

| Komponenten | Beispiel Nr. 1 (Vol.%) | Beispiel Nr. 2 (Vol.%) | Beispiel Nr. 3 (Vol.%) | Beispiel Nr. 4 (Vol.%) | Beispiel Nr. 5 (Vol.%) | Beispiel Nr. 6 (Vol.%) | Beispiel Nr. 7 (Vol.%) |
|---|---|---|---|---|---|---|---|
| $O_2$ | 0,2 | 1,0 | 2,8 | 0,15 | 4,9 | 3,4 | 1,0 |
| CO | 3,1 | 2,1 | 2,5 | 3,3 | 1,4 | 1,4 | 2,3 |
| $CO_2$ | 3,5 | 2,3 | 2,8 | 3,0 | 2,7 | 1,7 | 2,8 |
| $C_2H_4$ | 4,9 | 1,5 | 5,7 | 3,0 | 1.2 | 1,4 | 3,8 |
| Vinylchlorid | 0,022 | 0,0021 | 0,0185 | 0,0275 | 0,0215 | 0,0125 | 0,028 |
| $C_2H_5Cl$ | 0,074 | 0,041 | 0,043 | 0,09 | 0,072 | 0,075 | 0,059 |
| Leichtsieder | 0,005 | 0,004 | 0,004 | 0,012 | 0,0165 | 0,005 | 0,004 |
| 1,2-Dichlorethan (EDC) | 3,3 | 3,3 | 3,15 | 4,1 | 4,5 | 3,8 | 3,1 |
| Hochsieder | — | — | — | — | — | — | — |
| 1,1,2-Trichlorethan | 0,002 | 0,0015 | 0,004 | 0,006 | 0,006 | 0,0015 | 0,002 |
| $Cl_2$ im Abgas | frei | frei | frei | frei | frei | frei | frei |
| $Cl_2$ im Wasser | frei | frei | frei | frei | frei | frei | frei |
| $Cl_2$ im Roh-EDC | frei | frei | frei | frei | frei | frei | frei |

## Parentansprüche

1. Verfahren zur Herstellung von 1,2-Dichlorethan durch Umsetzung von Ethylen mit Chlor, Chlorwasserstoff und Sauerstoff oder Sauerstoff enthaltenden Inertgasen bei 180 bis 260°C und einem Druck von 0,09 bis 1,1 MPa in Gegenwart eines Kupfer enthaltenden Festbettkatalysators in einem Reaktionsraum, der heiz- und kühlbare Flächen enthält, wobei das gasförmige Reaktionsgemisch nach Durchströmen des Festbettketalysators aus dem Reaktionsraum abgeführt, gekühlt und nach bekannten Verfahren das gebildete 1,2-Dichlorethan abgetrennt wird, dadurch gekennzeichnet, dass in den Reaktionsraum vor Beginn des Katalysatorfestbettes Ethylen, Chlor sowie Sauerstoff oder Sauerstoff enthaltende Inertgase und entweder an der gleichen Stelle wie die vorgenannten Gase oder im ersten Drittel des Katalysatorfestbettes Chlorwasserstoff in folgenden Molverhältnissen eingeleitet werden: 1 mol $C_2H_4$/0,35 bis 0,7 mol $Cl_2$/0,7 bis 1,4 mol $HCl$/0,2 bis 0,6 mol $O_2$.

2. Verfahren nach Anspruch 1, dadurch gekennzeichnet, dass bei Temperaturen von 190 bis 240°C gearbeitet wird.

3. Verfahren nach einem der beiden Ansprüche 1 oder 2, dadurch gekennzeichnet, dass im Katalysatorfestbett die Konzentration des Kupfers in Strömungsrichtung der Gase von mindestens 1 Gew.% bis zu höchstens 20 Gew.%, jeweils bezogen auf das Gesamtgewicht des Festbettes, kontinuierlich oder in Stufen zunimmt.

4. Verfahren nach einem der Ansprüche 1 bis 3, dadurch gekennzeichnet, dass das Kupfer auf porösem Aluminiumoxid oder Siliciumdioxid oder Mischungen dieser beiden Oxide niedergeschlagen ist.

5. Verfahren nach einem der Ansprüche 1 bis 4, dadurch gekennzeichnet, dass der Festbettkatalysator neben Kupfer wenigstens eine der folgenden Verbindungen: Natrium-, Kalium-, Calcium- oder Magnesiumchlorid enthält.

6. Verfahren nach einem der Ansprüche 1 bis 5, dadurch gekennzeichnet, dass der Festbettkatalysator neben Kupfer Eisen in Mengen von 0,001 bis 1 Gew.%, bezogen auf das Gesamtgewicht des Festbettes, enthält.

7. Verfahren nach einem der Ansprüche 1 bis 6, dadurch gekennzeichnet, dass das Katalysatorfestbett zu 25 bis 75 Vol.%, bezogen auf das Volumen aller Teilchen des Festbettes, aus inerten Teilchen besteht, die bei 200°C eine Wärmeleitzahl von mindestens 400 $Jh^{-1}$ $cm^{-1}K^{-1}$ aufweisen.

8. Verfahren nach einem der Ansprüche 1 bis 7, dadurch gekennzeichnet, dass das Katalysatorfestbett einen freien Gasraum von 20 bis 45 Vol.% besitzt.

9. Verfahren nach einem der Ansprüche 1 bis 8, dadurch gekennzeichnet, dass die Gase in folgenden Molverhältnissen dem Reaktionsraum zugeführt werden: 1 mol $C_2H_4$/0,4 bis 0,55 mol $Cl_2$/0,9 bis 1,2 mol HCl/0,25 bis 0,4 mol $O_2$.

10. Verfahren nach einem der Ansprüche 1 bis 9, dadurch gekennzeichnet, dass auf 1 mol Ethylen 1,8 bis 1,99 mol Cl, berechnet aus der Summe von $Cl_2$ und HCl, eingesetzt werden.

11. Verfahren nach einem der Ansprüche 1 bis 10, dadurch gekennzeichnet, dass als Sauerstoff enthaltende Inertgase Luft, gegebenenfalls in Mischung mit Sauerstoff, eingesetzt werden.

## Revendications

1. Procédé de préparation du dichloro-1,2-éthane par réaction de l'éthylène avec le chlore, le chlorure d'hydrogène et l'oxygène, ou des gaz inertes contenant de l'oxygène, entre 180 et 260°C et sous une pression de 0,09 à 1,1 MPa en présence d'un catalyseur en lit fixe contenant du cuivre dans un espace de réaction qui contient des surfaces pouvant être chauffées et refroidies, le mélange gazeux de réaction étant, après avoir parcouru le catalyseur en lit fixe, retiré de l'espace de réaction, refroidi et le dichloro-1,2-éthane formé étant séparé par des procédés connus, procédé caractérisé en ce qu'on introduit dans l'espace de réaction, avant le début du lit fixe de catalyseur, l'éthylène, le chlore ainsi que l'oxygène, ou les gaz inertes contenant de l'oxygène, et, au même endroit que pour les gaz précités ou dans le premier tiers du lit fixe du catalyseur, le chlorure d'hydrogène, selon les rapports molaires suivants: 1 mol de $C_2H_4$/0,35 à 0,7 mol de $Cl_2$/0,7 à 1,4 mol de HCl/0,2 à 0,6 mol de $O_2$.

2. Procédé selon la revendication 1, caractérisé en ce qu'on travaille à des températures de 190 à 240°C.

3. Procédé selon l'une des revendications 1 ou 2, caractérisé en ce que, dans le lit fixe du catalyseur, la concentration du cuivre augmente, continuellement ou par étapes dans le sens de l'écoulement des gaz, de 1% en poids au minimum jusqu'à 20% en poids au maximum, chaque fois par rapport au poids total du lit fixe.

4. Procédé selon l'une quelconque des revendications 1 à 3, caractérisé en ce que le cuivre est déposé sur de l'oxyde d'aluminium ou du dioxyde de silicium poreux ou sur des mélanges de ces deux oxydes.

5. Procédé selon l'une quelconque des revendications 1 à 4, caractérisé en ce que en plus du cuivre, le catalyseur en lit fixe contient au moins l'un des composés suivants: du chlorure de sodium, de potassium, de calcium ou de magnésium.

6. Procédé selon l'une quelconque des revendications 1 à 5, caractérisé en ce que en plus du cuivre, le catalyseur en lit fixe contient du fer en des quantités de 0,001 à 1% en poids, par rapport au poids total du lit fixe.

7. Procédé selon l'une quelconque des revendications 1 à 6, caractérisé en ce que le lit fixe de catalyseur consiste, pour 25 à 75% en volume, par rapport au volume de toutes les particules du lit fixe, en des particules inertes qui présentent à 200°C un coefficient de conductibilité thermique d'au moins 400 Jh⁻¹ cm⁻¹ K⁻¹.

8. Procédé selon l'une quelconque des revendications 1 à 7, caractérisé en ce que le lit fixe du catalyseur présente un espace libre pour les gaz de 20 à 45% en volume.

9. Procédé selon l'une quelconque des revendications 1 à 8, caractérisé en ce qu'on achemine à l'espace de réaction les gaz selon les rapports molaires suivants: 1 mol de $C_2H_4$/0,4 à 0,55 mol de $Cl_2$/0,9 à 1,2 mol de HCl/0,25 à 0,4 mol de $O_2$.

10. Procédé selon l'une quelconque des revendications 1 à 9, caractérisé en ce que, pour 1 mol d'éthylène, on utilise 1,8 à 1,99 mol de Cl, quantité calculée à partir de la somme de $Cl_2$ et de HCl.

11. Procédé selon l'une quelconque des revendications 1 à 10, caractérisé en ce que, comme gaz inerte contenant de l'oxygène, on utilise de l'air, éventuellement en mélange avec de l'oxygène.

## Claims

1. A process for the manufacture of 1,2-dichlorethane by reaction of ethylene with chlorine, hydrogen chloride and oxygen or oxygen-containing inert gases, at 180 to 260°C and a pressure of from 0.09 to 1.1 MPa, in the presence of a copper-containing fixed bed catalyst, in a reaction zone containing surfaces capable of being heated and cooled, the gaseous reaction mixture being removed from the reaction zone after passage through the fixed bed catalyst, cooled, and the 1,2-dichlorethane formed being separated according to known methods, characterized by introducing into the reaction zone ethylene, chlorine and oxygen or oxygen-containing inert gases before the fixed catalyst bed, and hydrogen chloride either at the same place as said other gases or in the first third of the fixed catalyst bed at the latest, in the following quantitative ratio: 1 mol $C_2H_4$/0.35 to 0.7 mol $Cl_2$/0.7 to 1.4 mol HCl/0.2 to 0.6 mole $O_2$.

2. The process as claimed in claim 1, characterized by operating at temperatures of from 190 to 240°C.

3. The process as claimed in claim 1 or 2, characterized by increasing the concentration of copper in the fixed catalyst bed continuously or in steps in the direction of gas flow from at least 1 wt.% to 20 wt.% at most, each relative to the total weight of the fixed bed.

4. The process as claimed in one of claims 1 to 3, characterized by applying the copper by precipitation onto porous aluminum oxide or silicon dioxide or mixtures of these two oxides.

5. The process as claimed in one of claims 1 to 4, characterized in that the fixed bed catalyst contains in addition to copper at least one of the following compounds: sodium chloride, potassium chloride, calcium chloride or magnesium chloride.

6. The process as claimed in one of claims 1 to 5, characterized in that the fixed bed catalyst contains iron in amounts of from 0.001 to 1 wt.%, relative to the total weight of the fixed bed.

7. The process as claimed in one of claims 1 to 6, characterized in that the fixed catalyst bed consists of 25 to 75% by volume, relative to the volume of all particles of the fixed bed, of inert particles having a coefficient of heat conductivity at 200°C of at least 400 $Jh^{-1}$ $cm^{-1}K^{-1}$.

8. The process as claimed in one of claims 1 to 7, characterized in that the fixed catalyst bed has a free gas space of from 20 to 45% by volume.

9. The process as claimed in one of claims 1 to 8, characterized by introducing the gases into the reaction zone in the following molar ratio: 1 mol $C_2H_4$/0.4 to 0.55 mol $Cl_2$/0.9 to 1.2 mol HCl/0.25 to 0.4 mol $O_2$.

10. The process as claimed in one of claims 1 to 9, characterized by using from 1.8 to 1.99 mol of Cl/mol of ethylene, calculated on the sum of $Cl_2$ and HCl.

11. The process as claimed in one of claims 1 to 10, characterized by using air, optionally in admixture with oxygen, as oxygen-containing inert gas.